# EUROPEAN PATENT SPECIFICATION

(11) **EP 3 922 109 B1**
(45) Date of publication and mention of the grant of the patent: **20.05.2026**
(21) Application number: 20790935.9
(22) Date of filing: 09.04.2020
(51) Int. Cl.: A23L 27/30, C07H 15/24

(54) **COMPOSITION COMPRISING FRUCTOSE-TRANSFERRED STEVIOL GLYCOSIDE**
ZUSAMMENSETZUNG MIT FRUCTOSETRANSFERIERTEM STEVIOLGLYCOSID
COMPOSITION COMPRENANT UN GLYCOSIDE DE STÉVIOL TRANSFÉRÉ PAR FRUCTOSE

(30) Priority: 19.04.2019 KR 20190046300
(43) Date of publication of application: 15.12.2021
(73) Proprietor: CJ Cheiljedang Corporation, Seoul 04560 (KR)
(72) Inventor: KIM, Jungeun, Seoul 04560 (KR); YANG, Tae Joo, Seoul 04560 (KR); PARK, Sunghee, Seoul 04560 (KR); KANG, In Sung, Seoul 04560 (KR); KIM, Seong Bo, Seoul 04560 (KR); CHU, Sun, Seoul 04560 (KR)
(74) Representative: Icosa
(86) International application number: PCT/KR2020/004809
(87) International publication number: WO 2020/213891

(56) References cited:
- WO-A1-2017/093895
- JP-B2- H0 577 675
- KR-A- 950 002 868
- KR-B1- 101 199 821
- KR-B1- 101 767 606
- ISHIKAWA HIROSHI ET AL, AGRICULTURAL AND BIOLOGICAL CHEMISTRY, vol. 54, no. 12, 8 December 1990 (1990-12-08), JP, pages 3137 - 3143, XP055815553, ISSN: 0002-1369, DOI: 10.1080/00021369.1990.10870467
- KITAHATA S ET AL: "Production and properties of oligosaccharides synthesized from sucrose", 1 January 2000, GLYCOENZYMES, TOKYO : JAPAN SCIENTIFIC SOC. PRESS ; BASEL [U.A.] : KARGER, JP, PAGE(S) 227 - 239, ISBN: 978-4-7622-2942-8, XP008125557
- GERWIG GERRIT J. ET AL: "Advances in Carbohydrate Chemistry and Biochemistry", vol. 73, 1 January 2016 (2016-01-01), US, pages 1 - 72, XP055843071, ISBN: 978-0-12-809983-4, Retrieved from the Internet <URL:http://dx.doi.org/10.1016/bs.accb.2016.05.001> DOI: 10.1016/bs.accb.2016.05.001

## Description

### [Technical Field]

The present disclosure relates to a sweetener composition including a transfructosylated steviol glycoside.

### [Background Art]

As the World Health Organization (WHO) recommends lowering the amount of daily sugar intake due to concerns about disease (obesity) caused by sugar consumption, various policies aimed at reducing the amount of sugar intake are actively being discussed by the governments of developed countries. Therefore, as the need for developing various alternative sweeteners is increasing in the market, alternative sweeteners are continuously being developed and commercialized. As alternative sweeteners, these are the subject of continuous variation in the form of synthetic high-intensity sweeteners (*e.g.,* Saccharin, Aspartame, Sucralose, *etc*.)*,* synthetic sugar alcohols (*e.g.,* Maltitol and Xylitol), and high-intensity sweeteners (*e.g.,* Rebaudioside A and Liquorice). Nevertheless, due to concerns over the safety of synthetic sweeteners, customers' need for natural sweeteners has been steadily increasing; however, because of limitations to peculiar flavor properties of natural sweeteners (*i.e.,* off-smell and off-flavor), natural sweeteners cannot fully replace existing low-calorie and zero-calorie products based on synthetic sweeteners.

A natural high-intensity sweetener that has received considerable attention in recent years is stevia extracted from the leaves of *Stevia rebaudiana Bertoni.* Stevia is a natural material, the sweetness of which is 200 to 300 times that of sugar, and consists of Stevioside, Rebaudioside A, *etc.* Although Stevioside and Rebaudioside A show high sweetness, they have a strong bitter taste, which presents a limitation in use.

Methods to improve the sweetness of Stevioside and Rebaudioside A include a method for transferring a saccharide using an enzyme. As the method for transferring a saccharide using an enzyme, a method for transferring 1 to 12 glucose molecules to a steviol glycoside using CGTase is widely used in the art (Korean Patent Application No. 10-1991-0020769).
Ishikawa et al. (Production of Stevioside and Rubososide derivatives by transfuctosylation of β-fructofuranosidase, Agric. Biol. Chem. 54 (1990)) describe a method for producing transfructosylated steviol glycosides and transfructosylated rubusoside derivatives by a microorganism of *Arthrobacter* sp.. They further disclose that a fructose residue is regioselectively transferred to the 19-OH position of steviol glycosides by β-2,6-linkage.
Kitahata et al. (Production and properties of oligosaccharides synthesized from sucrose, Japan Scientific Soc. Press, 2000*)* describe a method for preparing fructooligosaccharides and further disclose that fructosyl stevioside is synthesized using the enzyme of *Arthrobacter* sp. K-1 and that a fructose residue is transferred to the hydroxyl group of the C-6 position of the glucose molecule of stevioside.
The scientific review of Gerwig Gerrit et al. (Stevia Glycosides, Advances in Carbohydrate Chemistry and Biochemistry, 2016) relates to chemical and enzymatic modifications of their carbohydrate moieties for the improvement of sweet tasting quality of stevia glycosides, and this review summarizes the structures of native steviol glycosides and the recent data of modifications of the carbohydrate moieties that have been published.
The patent application KR 950 002 868 relates to a method for producing fructosyl stevioside and a sweetener comprising the same as an active ingredient. Specifically, this application discloses β-2,6-fructosyl stevioside, in which fructose is transferred to the C-6 position of glucose that is bound to the C-19 of the stevioside backbone.
However, all these methods have a disadvantage in that all glucose molecules transferred to the steviol glycoside are degraded by intestinal microorganisms, increasing calories. Therefore, there is a need for a steviol glycoside wherein a saccharide other than glucose is transferred.

### [Disclosure]

### [Technical Problem]

Under such circumstances, the present inventors have completed the present disclosure by confirming that the transfructosylated steviol glycoside has an improved bitter taste compared to a steviol glycoside, and shows superior sweetness preference and overall preference.

### [Technical Solution]

An object of the present disclosure is a method to provide a sweetener composition including a transfructosylated steviol glycoside by fructosyltransferase derived from Arthrobacter globiformis, wherein the transfructosylated steviol glycoside is in the form in which fructose is linked to a 19-OH site of a steviol glycoside or to glucose conjugated thereto, wherein the steviol glycoside is stevioside or rebaudioside A.

Another object of the present disclosure is to provide a food composition including the sweetener composition.

Still another object of the present disclosure is to provide a method for improving the sweetness of a steviol glycoside, including converting a steviol glycoside into a transfructosylated steviol glycoside by fructosyltransferase derived from Arthrobacter globiformis, wherein the transfructosylated steviol glycoside is in the form in which fructose is linked to a 19-OH site of a steviol glycoside or to glucose conjugated thereto, wherein the steviol glycoside is stevioside or rebaudioside A.

### [Advantageous Effects]

The sweetener composition of the present disclosure includes a transfructosylated steviol glycoside, thereby having an improved bitter taste compared to a steviol glycoside and showing superior sweetness preference and overall preference, and thus can be used as a high-intensity sweetener.

### [Brief Description of Drawings]

Fig. 1 shows the results of sensory descriptive analysis of transfructosylated Rebaudioside A (RA-Fru) and Rebaudioside A (RA).
Fig. 2 shows the results of sensory descriptive analysis of transfructosylated Stevioside (STV-Fru) and Stevioside (STV).

### [Detailed Description of the Invention]

Hereinbelow, the present disclosure will be described in detail. Meanwhile, each of the explanations and exemplary embodiments disclosed herein can be applied to other explanations and exemplary embodiments. That is, all combinations of various factors disclosed herein belong to the scope of the present disclosure. Furthermore, the scope of the present disclosure should not be limited by the specific disclosure provided hereinbelow. Meanwhile, the terms defined herein can be identically applied to other aspects of the present disclosure.

In order to achieve the objects of the present disclosure, an aspect of the present disclosure may be to provide a transfructosylated steviol glycoside or a sweetener composition including the transfructosylated steviol glycoside by fructosyltransferase derived from Arthrobacter globiformis, wherein the transfructosylated steviol glycoside is in the form in which fructose is linked to a 19-OH site of a steviol glycoside or to glucose conjugated thereto, wherein the steviol glycoside is stevioside or rebaudioside A.

As used herein, the term "steviol glycoside" refers to a natural sweetener and may be represented by Chemical Formula 1 below.

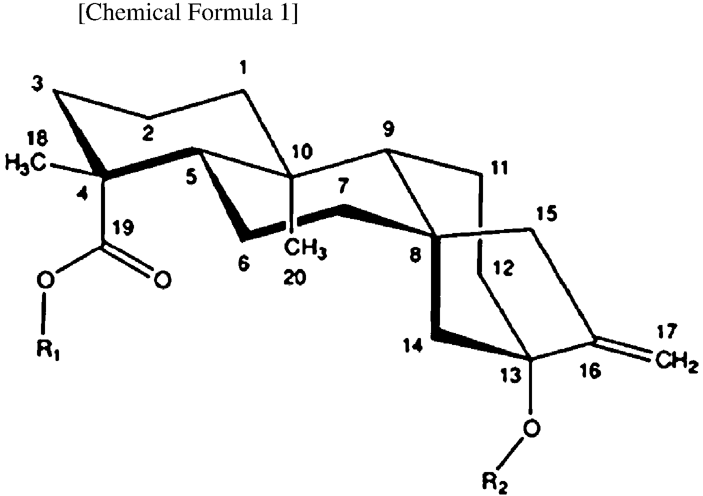

In Chemical Formula 1, at R₁, hydrogen (H) may be bound, or 1 to 3 glucose molecules may be bound via a β-bond; and at R₂, one molecule of glucose, xylose, or rhamnose may be bound via a β-bond, and 0 to 2 glucose molecules may be bound thereto via a β-bond, but these are not limited thereto.

The Stevioside is represented by Chemical Formula 2 below or Rebaudioside A is represented by Chemical Formula 3 below.

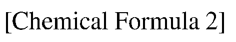

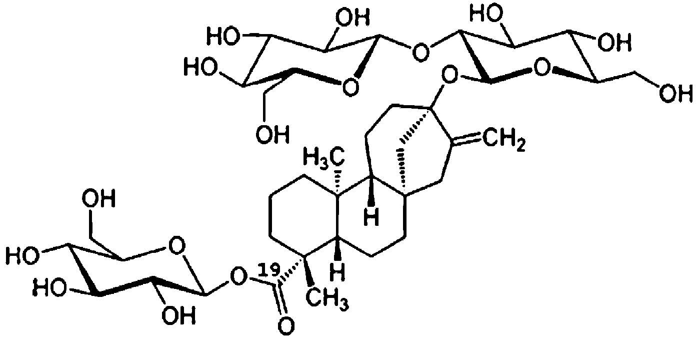

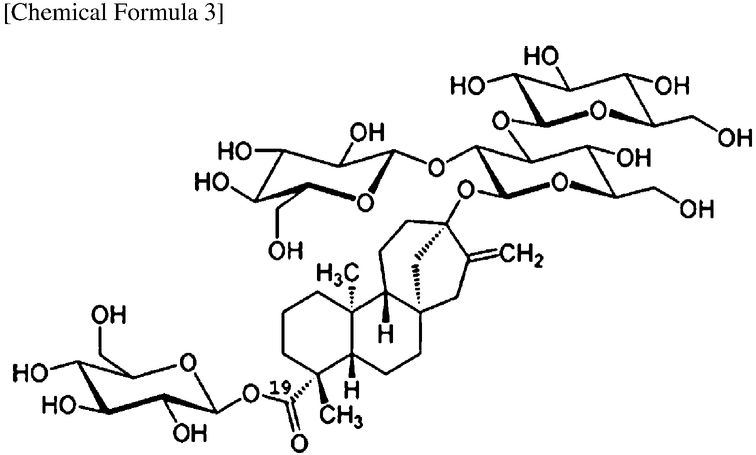

As used herein, the term "transfructosylated steviol glycoside" may refer to a compound in which fructose is linked (transferred) to the steviol glycoside.

The transfructosylated steviol glycoside is in the form in which fructose is linked to a 19-OH site of the steviol glycoside or to glucose conjugated thereto. Specifically, the transfructosylated steviol glycoside may be in the form in which fructose is linked to the steviol glycoside via a β-(2,6) bond.

Specifically, the transfructosylated steviol glycoside may be in the form in which 1 to 3 fructose molecules are linked to the steviol glycoside.

The transfructosylated steviol glycoside is a transfructosylated Stevioside or a transfructosylated Rebaudioside A. Accordingly, the sweetener composition of the present disclosure may include a transfructosylated Stevioside or a transfructosylated Rebaudioside A, or a transfructosylated Stevioside and a transfructosylated Rebaudioside A.

The sweetener composition including a transfructosylated Stevioside and a transfructosylated Rebaudioside A may contain 40 parts by weight or more of the transfructosylated Rebaudioside A based on 100 parts by weight of the total weight of the transfructosylated Stevioside and transfructosylated Rebaudioside A. Specifically, the composition may contain 50 parts by weight or more, more specifically 60 parts by weight or more, even more specifically 70 parts by weight or more, even more specifically 75 parts by weight or more of the transfructosylated Rebaudioside A.

Specifically, the transfructosylated steviol glycoside may have an improved bitter taste as compared to the stveiol glycoside. The improvement in bitter taste may mean a decrease in bitter taste.

In particular, the improvement in bitter taste may be evaluated when the transfructosylated steviol glycoside and the steviol glycoside have equivalent sweetness. Specifically, the sweetness may be the sweetness corresponding to 10 brix of sugar.

Specifically, the transfructosylated steviol glycoside may have an improved sweetness as compared to the steviol glycoside. The improvement in sweetness may be an increase in sweetness preference and overall preference.

In particular, the improvement in sweetness may be evaluated when the transfructosylated steviol glycoside and the steviol glycoside have equivalent sweetness. Specifically, the sweetness may be the sweetness corresponding to 10 brix of sugar.

Specifically, the transfructosylated steviol glycoside may be produced by reacting the steviol glycoside and a fructose donor in the presence of a fructosyltransferase, but for the purposes of the present disclosure, such reaction is not limited as long as the transfructosylated steviol glycoside can be obtained.

Specific nucleotide sequence of a gene encoding the fructosyltransferase and protein information thereof can be obtained from a known database such as GenBank of NCBI, *etc.* However, in addition to the above known sequences, any protein may be included as long as it exhibits the same effect of transferring fructose as the fructosyltransferase without limitation to its origin or sequence. Additionally, a homologous protein or a variant protein may also be included in the scope of the fructosyltransferase of the present disclosure.

The step of reacting the steviol glycoside with a fructose donor in the presence of the enzyme may be carried out at a pH of 3 to 8, specifically at a pH of 4 to 7, and more specifically at a pH of 5 to 6, but the pH range is not limited thereto.

Additionally, the above step may be carried out at 10°C to 60°C, specifically at 20°C to 50°C, and more specifically at 20°C to 40°C, but the temperature range is not limited thereto.

The sweetener composition of the present disclosure may be used for preparing sweeteners or providing sweetness to edible products. The sweetener composition of the present disclosure may be used as a sweetener for the purpose of cooking and/or processed foods. Additionally, the sweetener composition of the present disclosure may be used as a sweetener for pharmaceutical products in addition to the edible products, but is not limited thereto. Further, the sweetener composition of the present disclosure may further include a flavoring agent, a preservative, a stabilizer, an antioxidant, *etc.,* but is not limited thereto.

In order to achieve the objects of the present disclosure, another aspect of the present disclosure provides a food composition including the sweetener composition.

The food composition of the present disclosure may be prepared using methods commonly used in the art, and may be prepared by adding raw materials and ingredients commonly added in the art during preparation. Additionally, the food composition of the present disclosure uses a food as a raw material unlike generic drugs, and thus has no side effects that may occur during long-term administration thereof, and may be highly portable.

The food composition may contain the sweetener composition in an amount of 0.001 to 25% by weight, specifically 0.01 to 20% by weight, more specifically 0.01 to 10% by weight, based on the total weight of the composition, but is not limited thereto.

In order to achieve the objects of the present disclosure, still another aspect of the present disclosure provides a method for improving the sweetness of a steviol glycoside, including converting a steviol glycoside into a transfructosylated steviol glycoside by fructosyltransferase derived from Arthrobacter globiformis, wherein the transfructosylated steviol glycoside is in the form in which fructose is linked to a 19-OH site of a steviol glycoside or to glucose conjugated thereto, wherein the steviol glycoside is stevioside or rebaudioside A.

The "steviol glycoside" and the "transfructosylated steviol glycoside" are as described above.

For the purpose of the present disclosure, the conversion is not limited by its method as long as the transfructosylated steviol glycoside can be obtained from the steviol glycoside.

Specifically, the conversion may include reacting the steviol glycoside with a fructose donor in the presence of a fructosyltransferase, but is not limited thereto.

The fructose donor may be any oligomer, polymer, or cyclic form of fructose which can be reacted in the presence of a fructosyltransferase such that one or more fructose molecules can be transferred to the steviol glycoside, and specifically, it may be sugar, but is not limited thereto.

Specific nucleotide sequence of a gene encoding the fructosyltransferase and protein information thereof can be obtained from a known database such as GenBank of NCBI, *etc.* However, in addition to the above known sequences, any sequence may be included as long as it exhibits the same effect of transferring fructose as the fructosyltransferase without limitation to its origin or sequence, and a homologous protein or a variant protein may also be included in the scope of the fructosyltransferase of the present disclosure.

The step of reacting the steviol glycoside with a fructose donor may be carried out at a pH of 3 to 8, specifically at a pH of 4 to 7, and more specifically at a pH of 5 to 6, but the pH range is not limited thereto.

Additionally, the above step may be carried out at 10°C to 60°C, specifically at 20°C to 50°C, and more specifically at 20°C to 40°C, but the temperature range is not limited thereto.

As used herein, the term "improvement in sweetness" may mean an improvement in sweetness preference or overall preference as the bitter taste or the off-smell/off-flavor is reduced.

### [Detailed Description of the Invention]

Hereinbelow, the present disclosure will be described in detail with accompanying exemplary embodiments. However, the exemplary embodiments disclosed herein are only for illustrative purposes and should not be construed as limiting the scope of the present disclosure.

### Preparation Example 1: Preparation of Transfructosylated Stevioside and Transfructosylated Rebaudioside A

5% Stevioside (Carbosynth) or 5% Rebaudioside A (Purecircle) and 20% white sugar (CJ CheilJedang) were dissolved in 50 mM sodium acetate buffer, and then a fructosyltransferase derived from *Arthrobacter globiformis* was added thereto, and the mixture was reacted at 40°C for 24 hours. Subsequently, the mixture was adsorbed onto an adsorption resin (HP-20) and desorbed with 20% ethanol. Thereafter, the desorbed solution was concentrated under reduced pressure and dried to prepare a sample.

The fructosyltransferase transfers fructose from sugar. In particular, the resulting transfructosylated product is in the form in which 1 to 3 fructose molecules are linked (transferred) to the 19-OH position of Stevioside or Ribaudioside A or to glucose conjugated thereto, by a linkage via a β-(2,6) bond.

The structure of the thus-prepared transfructosylated Stevioside and transfructosylated Rebaudioside A was analyzed by ¹H/¹³C NMR, Homonuclear Correlation Spectroscopy (COSY), Total Correlation Spectroscopy (TOCSY), Heteronuclear Single-Quantum Coherence (HSQC), and Heteronuclear Multiple-Bond Correlation (HMBC), and the results (¹H/¹³C NMR, COSY and HMBC) are shown in Tables 1 and 2.

Additionally, upon confirmation of the structure of the transfructosylated Stevioside and the transfructosylated Rebaudioside A, the results revealed that the transfructosylated Stevioside was found to be 13-[(2-O-β-D-Glucopyranosyl-α-D-glucopyranosyl)oxy]kaur-16-en-18-oic acid 6-O-β-D-fructofuranose-β-D-glucopyranosyl ester, and the transfructosylated Rebaudioside A was found to be 13-[(2-O-β-D-glucopyranosyl-3-O-β-D-glucopyranosyl-β-D-glucopyranosyl)oxy]ent-kaur-16-en-19-oic acid 6-O-β-D-fructofuranose-β-D-glucopyranosyl ester.

The transfructosylated Stevioside and the transfructosylated Rebaudioside A were represented by Chemical Formula 4 and Chemical Formula 5, respectively.

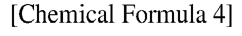

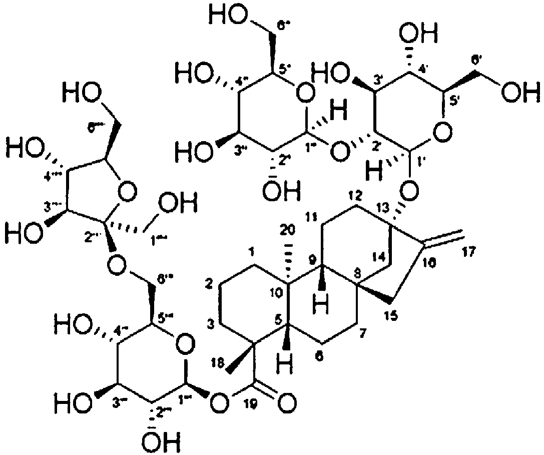

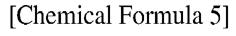

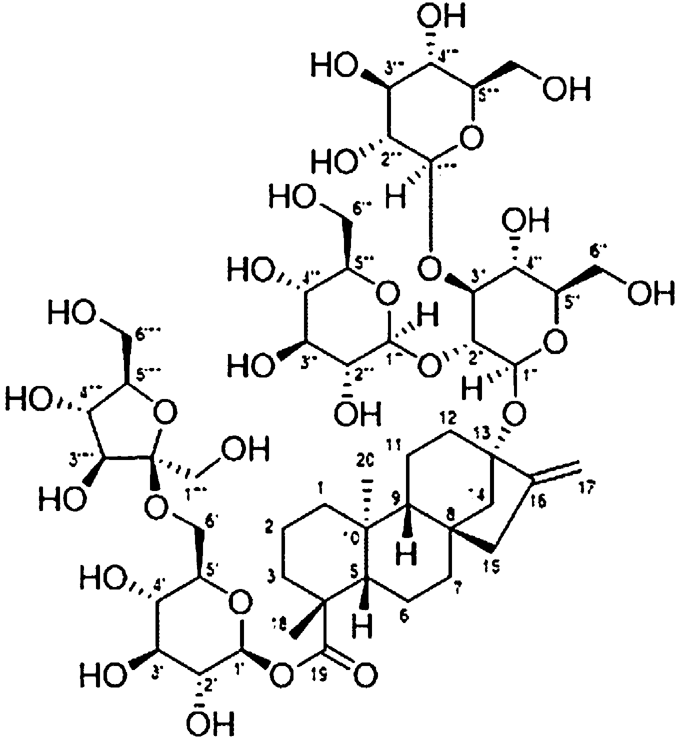

**[Table 1]**

| Position | · _{H} mult. (*J* in Hz) | ·_{C} mult. | | HMBC Correlation | COSY correlation | Key ROESY correlation |
|---|---|---|---|---|---|---|
| | | | | | | |
| 1 | 0.77 br m / 1.81 br m | 40.3 | CH₂ | | 1.81 / 0.77 | |
| 2 | 1.37 br m / 1.78 br m | 18.7 | CH₂ | | 1.78 / 1.37 | |
| 3 | 1.00 br m / 2.09 br m | 37.5 | CH₂ | 56.8 / 40.3, 43.8 | 1.78, 2.09 / 1.00 | / 1.18 |
| 4 | | 43.8 | C | | | |
| 5 | 1.07 br s | 56.8 | CH | 21.4, 28.0, 39.3, 40.9, 43.8, 178.6 | 1.80 | |
| 6 | 1.80 br m | 21.4 | CH₂ | | 1.07 | 3.40 |
| 7 | 1.35 br m / 1.52 br m | 40.9 | CH₂ | /56.8, 42.1 | 1.52 / 1.35 | 1.18 |
| 8 | | 42.1 | C | | | |
| 9 | 0.90 br s | 53.4 | CH | 20.3, 36.1, 39.3, 42.1, 44.2, 47.0 | 1.55 | |
| 10 | | 39.3 | C | | | |
| 11 | 1.55 br m / 1.75 br m | 20.3 | CH₂ | 39.3, 53.4 / 39.3, 42.1, 86.9 | 1.90, 1.75 / 1.55 | |
| 12 | 1.47 br m / 1.90 br m | 36.1 | CH₂ | | 1.90 / 1.47 | |
| 13 | | 86.9 | C | | | |
| 14 | 1.40 br d (10.0) | 44.2 | | 36.1, 42.1, 53.4, 86.9 | 2.11 | |
| | 2.11 br m | | | 36.1, 47.0 | 1.40 | 0.83, 3.22 |
| 15 | 1.96 br d (17.0) | 47.0 | CH₂ | 42.1, 44.2, 53.4, 86.9, 153.0 | 2.12 | |
| | 2.12 br d (17.0) | | | 42.1, 53.4, 86.9, 104.8,153.0 | 1.96 | |
| 16 | | 153.0 | C | | | |
| 17 | 4.84 br s / 5.08 br s | 104.8 | CH₂ | | 2.12 / 1.96, 2.12 | / 3.22, 3.31 |
| 18 | 1.18 s | 28.0 | CH₃ | 37.5, 43.8, 56.8, 178.6 | | 1.80, 2.09, 3.40, 3.45, 3.65, 4.01, 5.34 |
| 19 | | 178.6 | C | | | |
| 20 | 0.83 s | 15.1 | CH₃ | 39.3, 40.3, 53.4, 56.8 | | 1.78, 2.11, 3.22, 3.25, 5.34 |
| 1' | 4.65 br d (7.5) | 95.8 | CH | 86.9 | 3.46 | 3.26, 3.57 |
| 2' | 3.46 br m | 80.6 | CH | 75.6, 95.8, 103.1 | 3.57, 4.65 | |
| 3' | 3.57 br m | 75.6 | CH | 69.6, 80.6, 95.8w | 3.33, 3.46 | |
| 4' | 3.33 br m | 69.6 | CH | 60.7, 75.6 | 3.26, 3.57 | |
| 5' | 3.26 br m | 75.6 | CH | 69.6 | 3.33, 3.62 | |
| 6' | 3.62 br m / 3.76 br m | 60.7 | CH₂ | | 3.26, 3.76 / 3.62 | |
| 1" | 4.62 d (7.5) | 103.1 | CH | 80.6 | 3.22 | 3.46, 3.41, 3.31, |
| 2" | 3.22 t (7.5) | 74.3 | CH | 75.6, 103.1 | 3.41, 4.62 | 0.83 |
| 3" | 3.41 br m | 75.6 | CH | 69.8, 74.3 | 3.22, 3.25 | |
| 4" | 3.25 br m | 69.8 | CH | 61.2, 75.6, 76.3 | 3.31, 3.41 | 0.83 |
| 5" | 3.31 br m | 76.3 | CH | 69.8 | 3.25, 3.59 | |
| 6" | 3.59 br m / 3.78 br m | 61.2 | CH₂ | | 3.31, 3.78 / 3.59 | |
| 1‴ | 5.34 d (8.0) | 94.1 | CH | 178.6, 75.8 | 3.40 | 0.83, 1.18, 3.45, 3.54 |
| 2‴ | 3.40 t (8.0) | 71.9 | CH | 94.1, 76.0 | 3.45, 5.34 | 1.18 |
| 3‴ | 3.45 br m | 76.0 | CH | 68.8 | 3.40 | 1.18 |
| 4‴ | 3.45 br m | 68.8 | CH | 60.1, 71.9, 76.0 | | |
| 5‴ | 3.54 br m | 75.6 | CH | 68.8 | 3.65 | |
| 6‴ | 3.65 br m | 60.1 | CH₂ | 75.6, **103.6** | 3.54 | / 3.54, 3.65 |
| | 3.87 br m | | | 68.8, 75.6, **103.6** | | |
| 1ʺʺ | 3.55 br m / 3.63 br m | 60.0 | CH₂ | 77.0, 103.6, | | |
| 2ʺʺ | | 103.6 | CH | | | |
| 3ʺʺ | 4.07 d (8.5) | 77.0 | CH | 60.0, 74.5, 103.6 | 4.01 | 3.55, 3.63, 3.76, |
| 4ʺʺ | 4.01 t (8.5) | 74.5 | CH | 62.5, 77.0, 81.4 | 3.76, 4.07 | 1.18, 3.53, 3.65, 3.76 |
| 5ʺʺ | 3.76 | 81.4 | CH | 74.5, 103.6 | 3.53, 4.01 | |
| 6ʺʺ | 3.53 br m / 3.65 br m | 62.5 | CH₂ | 74.5, 81.4 / 74.5 | 3.65, 3.76 / 3.53 | |

**[Table 2]**

| **No.** | **d_{H} mult. (*J* in Hz)** | **d_{C} mult** | | **HMBC Correlation** | **COSY corr.** | **Key ROESY corr.** |
|---|---|---|---|---|---|---|
| | | | | | | |
| 1 | 0.76 br m | 40.3 | CH₂ | 15.0 | 1.37^{w}, 1.78, 1.81 | |
| 2 | 1.37 br m | 18.7 | CH₂ | | 1.78 | |
| 3 | 0.99 br m | 37.6 | CH₂ | | 1.78, 2.08 | |
| 4 | | 43.8 | C | | | |
| 5 | 1.05 br m | 56.9 | CH | 15.0, 21.4, 28.0, 39.3, 40.9, | 1.81 | |
| 6 | 1.81 br m | 21.4 | CH₂ | | 1.35 | 3.40 |
| 7 | 1.35 br m | 40.9 | CH₂ | | 1.53 | |
| 8 | | 42.0 | C | | | |
| 9 | 0.89 br s | 53.5 | CH | 15.0, 20.3, 36.3, 39.3, 42.0, | 1.54 | |
| 10 | | 39.3 | C | | | |
| 11 | 1.54 br m | 20.3 | CH₂ | 42.0, 53.5, 87.1 | 1.75, 1.89 | |
| 12 | 1.46 br m | 36.3 | CH₂ | | 1.89 | |
| 13 | | 87.1 | C | | | |
| 14 | 1.43 br m | 44.0 | | 36.3, 42.0, 53.5, 87.1, | 2.09 | |
| 15 | 1.95 br d (16.0) | 47.1 | CH₂ | 44.0 | 2.14 | |
| 16 | | 152.9 | C | | | |
| 17 | 4.85 br s | 104.8 | CH₂ | 47.1, 87.1, 152.9 | 2.14 | |
| 18 | 1.17 s | 28.0 | CH₃ | 37.6, 43.8, 56.9, 178.4 | | 1.81, 3.40 |
| 19 | | 178.4 | C | | | |
| 20 | 0.83 s | 15.0 | CH₃ | 39.3, 40.3, 53.5, 56.9 | | 3.17, 3.41, 5.37 |
| 1' | 5.37 d (8.0) | 94.1 | CH | 75.2, 178.4 | 3.40 | 3.40, 3.47, 3.66 |
| 2' | 3.40 br m | 71.9 | CH | 76.3, 94.1 | 3.47, 5.37 | 1.17, 1.81 |
| 3' | 3.47 br m | 76.3 | CH | 68.9, 71.9, | 3.40 | |
| 4' | 3.48 br m | 68.9 | CH | 65.2, 75.2, 76.3 | 3.66 | |
| 5' | 3.66 br m | 75.2 | CH | | 3.48, 3.64 | |
| 6' | 3.64 br m | 65.2 | CH₂ | **97.8** | 3.87 | |
| 1" | 4.66 ovlp solv. | 95.9 | CH | 75.2, 87.1 | 3.65 | |
| 2" | 3.65 br m | 78.6 | CH | 85.1, 95.9, **102.11** | 4.66 | |
| 3" | 3.80 br m | 85.1 | CH | 68.5, 78.6, **102.20** | 3.42 | 3.30, 3.70, |
| 4'' | 3.42 br m | 68.5 | CH | 60.8, 75.2, 85.1 | 3.29 ^{~} 3.33,3.80 | |
| 5'' | 3.30 br m | 75.2 | CH | | 3.64 | 3.80 |
| 6'' | 3.64 br m | 60.8 | CH₂ | | 3.30, 3.78 | |
| 1‴ | 4.77 br d (8.0) | 102.1 | CH | 75,9, 76.5, **78.6** | 3.17 | 3.31, 3.37, 3.65 |
| 2‴ | 3.17 br m | 74.1 | CH | 75.9, 120.1 | 3.37, 4.77 | 1.46, 2.09 |
| 3‴ | 3.37 br m | 75.9 | CH | 70.2, 74.1 | 3.17 | |
| 4‴ | 3.18 br m | 70.3 | CH | 61.5, 75.9, 76.5 | 3.31 | |
| 5‴ | 3.31 br m | 76.5 | CH | 61.5 | 3.18, 3.55 | |
| 6‴ | 3.55 br m* | 61.5 | CH₂ | 76.5 | 3.80 | |
| 1ʺʺ | 4.70 ovlp solv. | 102.2 | CH | 75.8, 76.1, **85.1,** | 3.29 | |
| 2ʺʺ | 3.29 br m | 73.3 | CH | 75.8, 102.2 | 3.42, 4.70 | |
| 3ʺʺ | 3.42 br m | 75.8 | CH | 69.5, 73.3 | 3.29 ^{~} 3.33 | |
| 4ʺʺ | 3.33 br m | 69.5 | CH | 61.6, 75.8, 76.1 | 3.42 | |
| 5ʺʺ | 3.41 br m | 76.1 | CH | | 3.63 | |
| 6ʺʺ | 3.63 br m | 60.6 | CH₂ | 76.3 | 3.41, 3.83 | |
| 1‴ʺ | 4.83 br d (3.0) | 97.8 | CH | **65.2,** 71.7, 73.1 | 3.45 | 3.45, 3.64 |
| 2‴ʺ | 3.45 br m | 71.4 | CH | 97.8, | 3.63, 4.83 | |
| 3‴ʺ | 3.63 br m | 73.1 | CH | 69.3, 71.4 | 3.34, 3.45 | |
| 4‴ʺ | 3.34 br m | 69.3 | CH | 60.3, 71.7, 73.1 | 3.59, 3.63 | |
| 5‴ʺ | 3.59 br m | 71.7 | CH | | 3.34, 3.68 | |
| 6‴ʺ | 3.68 br m* | 60.3 | CH₂ | | 3.59, 3.71 | |

### Example 1: Sensory Descriptive Analysis

The transfructosylated Stevioside (STV-Fru) and the transfructosylated Rebaudioside A (RA-Fru) prepared in Preparation Example 1 were diluted to the sweetness equivalent to Stevioside (STV) and Rebaudioside A (RA) (sweetness based on 10 brix of sugar), respectively, and then presented to experts (n = 15) for descriptive analysis.

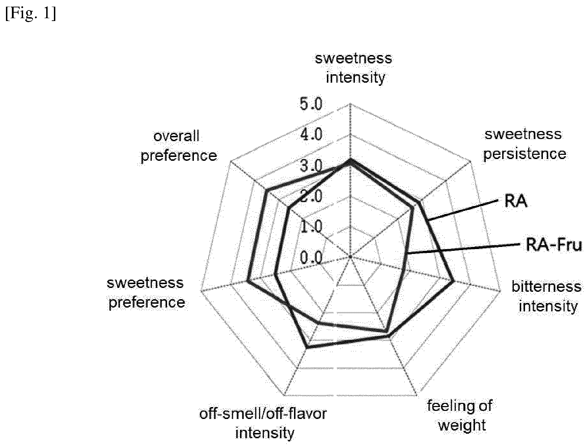

The results of comparing the transfructosylated Rebaudioside A (RA-Fru) and Rebaudioside A (RA) are shown in Table 3 and Fig. 1.

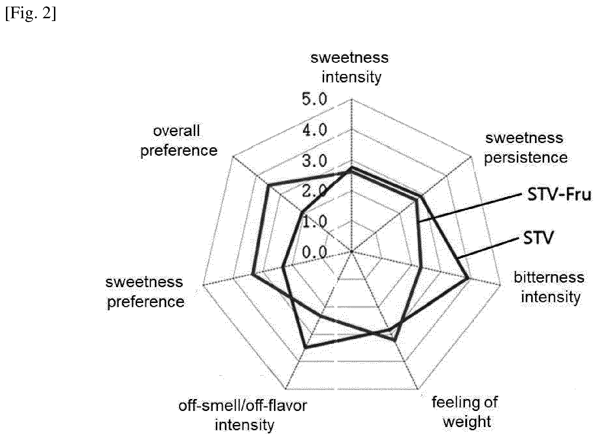

The results of comparing the transfructosylated Stevioside (STV-Fru) and Stevioside (STV) are shown in Table 4 and Fig. 2.

**[Table 3]**

| | RA | RA-Fru | p-value |
|---|---|---|---|
| Sweetness Intensity | 3.2 | 3.1 | 0.70 |
| Sweetness Persistence | 2.9 | 2.6 | 0.41 |
| Bitterness Intensity | 3.4 | 1.8 | 0.00 |
| Feeling of Weight | 2.9 | 2.7 | 0.49 |
| Off-smell/Off flavor Intensity | 3.3 | 2.4 | 0.01 |
| Sweetness Preference | 2.5 | 3.4 | 0.00 |
| Overall Preference | 2.6 | 3.5 | 0.01 |

**[Table 4]**

| | STV | STV-Fru | p-value |
|---|---|---|---|
| Sweetness Intensity | 2.8 | 2.6 | 0.64 |
| Sweetness Persistence | 2.9 | 2.7 | 0.72 |
| Bitterness Intensity | 3.9 | 2.3 | 0.00 |
| Feeling of Weight | 2.8 | 3.2 | 0.35 |
| Off-smell/Off flavor Intensity | 3.5 | 2.3 | 0.00 |
| Sweetness Preference | 2.3 | 3.3 | 0.00 |
| Overall Preference | 2.1 | 3.5 | 0.00 |

As shown in Tables 3 and 4, it can be found that the transfructosylated Rebaudioside A and the transfructosylated Stevioside (STV-Fru) have significantly low bitterness intensity and off-smell/off flavor intensity and significantly high sweetness preference and overall preference, as compared to Rebaudioside A (RA) and Stevioside (STV), respectively.

### Example 2: Sensory Preference Evaluation

Prior to sensory evaluation, four samples of Example 1 (STV, STV-Fru, RA, RA-Fru) were diluted to the sweetness equivalent to 10% (w/w) or 10 brix of sugar, and the sensory evaluation was carried out on the four samples.

The evaluation was proceeded as follow: 15 panels were given the four samples and required to evaluate the sweetness preference and the overall preference of the four samples on a 9-point scale (1= very much dislike, 5= neither dislike nor like, 9= very much like).

Then, the statistical analysis was converted to a 5-point scale to verify the significant difference through paired T tests (reliability level of 95%).

### (1) Comparison between STV and STV-Fru

**[Table 5]**

| Sensory Attributes | STV | STV-Fru | P-value |
|---|---|---|---|
| Sweetness Preference | 2.13 | 3.14 | 0.0002 |
| Overall Preference | 2.02 | 3.02 | 0.001 |

Table 5 shows that the transfructosylated Stevioside (STV-Fru) has excellent sweetness preference and overall preference as compared to Stevioside (STV).

### (2) Comparison between RA and RA-Fru

**[Table 6]**

| Sensory Attributes | RA | RA-Fru | P-value |
|---|---|---|---|
| Sweetness Preference | 2.51 | 3.42 | 0.0002 |
| Overall Preference | 2.72 | 3.73 | 0.00001 |

Table 6 shows that the transfructosylated Rebaudioside A (RA-Fru) has excellent sweetness preference and overall preference as compared to Rebaudioside A (RA).

### Preparation Example 2. Preparation of Mixture Comprising Transfructosylated Stevioside and Transfructosylated Rebaudioside A

A mixture of 5% Stevioside (Carbosynth) and 5% Rebaudioside A (Purecircle) (where the ratio of Rebaudioside A in the mixture was 40, 60, 80% by weight, respectively) and 20% white sugar (CJ CheilJedang) were dissolved in 50 mM sodium acetate buffer, and then a mixture comprising the transfructosylated Stevioside and the transfructosylated Rebaudioside A was prepared by the same manner as in Preparation Example 1. The ratio of the transfructosylated Stevioside to the transfructosylated Rebaudioside A in the thus-prepared mixture was maintained at the same level as the ratio of Stevioside to Rebaudioside A in the raw material.

The embodiments described above are considered to be illustrative in all respects and not restrictive. Furthermore, the scope of the present disclosure is defined by the appended claims rather than the detailed description, and it should be understood that all modifications or variations derived from the meanings and scope of the present disclosure and equivalents thereof are included in the scope of the appended claims.

## Claims

1. A method for preparing a sweetener composition, comprising converting a steviol glycoside into a transfructosylated steviol glycoside by fructosyltransferase derived from Arthrobacter globiformis,
wherein the transfructosylated steviol glycoside is in the form in which fructose is linked to a 19-OH site of a steviol glycoside or to glucose conjugated thereto,
wherein the steviol glycoside is stevioside or rebaudioside A.

2. The method for preparing the sweetener composition of claim 1, wherein the transfructosylated steviol glycoside is in the form in which fructose is linked to the steviol glycoside via a β-(2,6) bond.

3. The method for preparing the sweetener composition of claim 1, wherein the transfructosylated steviol glycoside is in the form in which 1 to 3 fructose molecules are linked to the steviol glycoside.

4. The method for preparing the sweetener composition of claim 1, wherein the transfructosylated steviol glycoside has an improved bitter taste compared to the steviol glycoside.

5. The method for preparing the sweetener composition of claim 1, wherein the transfructosylated steviol glycoside has improved sweetness compared to the steviol glycoside.

6. A method for improving the sweetness of a steviol glycoside, comprising converting a steviol glycoside into a transfructosylated steviol glycoside by fructosyltransferase derived from Arthrobacter globiformis,
wherein the transfructosylated steviol glycoside is in the form in which fructose is linked to a 19-OH site of the steviol glycoside or to glucose conjugated thereto,
wherein the steviol glycoside is stevioside or rebaudioside A.

7. The method of claim 6, wherein the transfructosylated steviol glycoside is in the form in which fructose is linked to the steviol glycoside via a β-(2,6) bond.

8. The method of claim 6, wherein the transfructosylated steviol glycoside is in the form in which 1 to 3 fructose molecules are linked to the steviol glycoside.

## Patentansprüche

1. Verfahren zur Herstellung einer Süßungsmittelzusammensetzung, umfassend Umwandeln eines Steviolglykosids in ein transfruktosyliertes Steviolglykosid durch Fructosyltransferase, welche aus Arthrobacter globiformis gewonnen wird,
wobei das transfruktosylierte Steviolglykosid in der Form vorliegt, in welcher Fructose an eine 19-OH-Site eines Steviolglykosids oder an damit konjugierte Glukose gekoppelt ist,
wobei das Steviolglykosid Steviosid oder Rebaudiosid A ist.

2. Verfahren zur Herstellung der Süßungsmittelzusammensetzung nach Anspruch 1, wobei das transfruktosylierte Steviolglykosid in der Form vorliegt, in welcher Fructose über eine β-(2,6)-Bindung an das Steviolglykosid gekoppelt ist.

3. Verfahren zur Herstellung der Süßungsmittelzusammensetzung nach Anspruch 1, wobei das transfruktosylierte Steviolglykosid in der Form vorliegt, in der 1 bis 3 Fruktosemoleküle an das Steviolglykosid gekoppelt sind.

4. Verfahren zur Zubereitung der Süßungsmittelzusammensetzung nach Anspruch 1, wobei das transfruktosylierte Steviolglykosid im Vergleich zu dem Steviolglykosid einen verbesserten bitteren Geschmack aufweist.

5. Verfahren zur Herstellung der Süßungsmittelzusammensetzung nach Anspruch 1, wobei das transfruktosylierte Steviolglykosid im Vergleich zu dem Steviolglykosid verbesserte Süße aufweist.

6. Verfahren zur Verbesserung der Süße eines Steviolglykosids, umfassend Umwandeln eines Steviolglykosids in ein transfruktosyliertes Steviolglykosid durch Fructosyltransferase aus Arthrobacter globiformis,
wobei das transfruktosylierte Steviolglykosid in der Form vorliegt, in welcher Fruktose an eine 19-OH-Site des Steviolglykosids oder an damit konjugierte Glukose gekoppelt ist,
wobei das Steviolglykosid Steviosid oder Rebaudiosid A ist.

7. Verfahren nach Anspruch 6, wobei das transfruktosylierte Steviolglycosid in der Form vorliegt, in welcher Fructose über eine β-(2,6)-Bindung an das Steviolglycosid gekoppelt ist.

8. Verfahren nach Anspruch 6, wobei das transfruktosylierte Steviolglykosid in der Form vorliegt, in welcher 1 bis 3 Fruktosemoleküle an das Steviolglykosid gekoppelt sind.

## Revendications

1. Un procédé de préparation d'une composition édulcorante, comprenant la conversion d'un glycoside de stéviol en un glycoside de stéviol transfructosylé par une fructosyltransférase dérivée d'Arthrobacter globiformis,
dans lequel le glycoside de stéviol transfructosylé est sous une forme dans laquelle le fructose est lié à un site 19-OH d'un glycoside de stéviol ou à du glucose lui étant conjugué,
dans lequel le glycoside de stéviol est du stévioside ou du rébaudioside A.

2. Le procédé de préparation de la composition édulcorante selon la revendication 1, dans lequel le glycoside de stéviol transfructosylé est sous une forme dans laquelle le fructose est lié au glycoside de stéviol via une liaison β-(2,6).

3. Le procédé de préparation de la composition édulcorante selon la revendication 1, dans lequel le glycoside de stéviol transfructosylé est sous une forme dans laquelle 1 à 3 molécules de fructose sont liées au glycoside de stéviol.

4. Le procédé de préparation de la composition édulcorante selon la revendication 1, dans lequel le glycoside de stéviol transfructosylé présente un goût amer amélioré par rapport au glycoside de stéviol.

5. Le procédé de préparation de la composition édulcorante selon la revendication 1, dans lequel le glycoside de stéviol transfructosylé présente une douceur améliorée par rapport au glycoside de stéviol.

6. Un procédé d'amélioration de la douceur d'un glycoside de stéviol, comprenant la conversion d'un glycoside de stéviol en un glycoside de stéviol transfructosylé par une fructosyltransférase dérivée d'Arthrobacter globiformis,
dans lequel le glycoside de stéviol transfructosylé est sous une forme dans laquelle le fructose est lié à un site 19-OH du glycoside de stéviol ou à du glucose lui étant conjugué,
dans lequel le glycoside de stéviol est du stévioside ou du rébaudioside A.

7. Le procédé selon la revendication 6, dans lequel le glycoside de stéviol transfructosylé est sous une forme dans laquelle le fructose est lié au glycoside de stéviol via une liaison β-(2,6).

8. Le procédé selon la revendication 6, dans lequel le glycoside de stéviol transfructosylé est sous une forme dans laquelle 1 à 3 molécules de fructose sont liées au glycoside de stéviol.
